# EUROPEAN PATENT APPLICATION

(11) **EP 3 485 801 A1**
(43) Date of publication of application: **22.05.2019**
(21) Application number: 17202470.5
(22) Date of filing: 19.11.2017
(51) Int. Cl.: A61B 5/00, A61B 5/145, A61B 5/1486

(54) **IMPLANTABLE INTEGRATED SENSOR DEVICE**

(71) Applicant: Indigo Diabetes N.V., 9052 Gent (BE)
(72) Inventor: ORDONEZ ORELLANA, Juan Sebastian, 9052 Gent (BE); ESPEEL, Pieter, 9052 Gent (BE)
(74) Representative: DenK iP

(57) **Abstract**

In a first aspect, the present invention relates to a sensor system, comprising an integrated sensor device (200), comprising a first integrated sensing element (410), configured for sensing a concentration of an analyte and a second integrated sensing element (420), configured for sensing an environmental variable. The sensor system also comprises a means for receiving and processing data, optionally partially or completely comprised in the integrated sensor device (200), and programmed for receiving an analyte concentration signal from the first integrated sensing element (410), and an environmental variable signal from the second integrated sensor element (420). The means for receiving and processing data is furthermore programmed for calibrating an interpretation of the received analyte concentration signal based on the received environmental variable signal.

## Description

### Technical field of the invention

The present invention relates to the field of integrated sensor devices and in particular to such integrated sensor devices adapted to calibrate to or modify a sensing environment.

### Background of the invention

Implanted sensor devices for measuring a target analyte (e.g. a biomarker) in a living tissue are known. For detection, they rely on the ability of the target analyte to diffuse towards the sensor. Typically, this involves the diffusion of the analyte from the capillaries to the interstitial fluid in the tissue and subsequent diffusion through the tissue to reach the sensor.

The diffusion properties of an analyte depend on e.g. its concentration in solution (e.g. in interstitial fluid). Furthermore, also the permeability properties of the tissue will depend on e.g. the amount of liquid that is present. The sensor output will thus depend on the hydration levels of the biological environment, as this will affect diffusion behaviour of the analyte, which translates to the measuring response of the sensor, and the concentration of analyte present in the sensing area, which translates to the signal intensity measured by the sensor.

Additionally, implanted sensor devices undergo encapsulation by fibrotic tissue after implantation. This is a natural response of the body to the incorporation of a foreign body into the tissue which was not destroyed and consumed and is part of the healing mechanism of the body after the medical intervention (i.e. implantation of the sensor device). The thickness and density of fibrotic tissue depend strongly on a multitude of parameters, e.g. the material that is implanted, the shape, the physical and chemical properties, the immune parameters of the targeted biology, the location of implantation, the extent of tissue damage due to the medical intervention, etc. The quality and amount of fibrotic tissue in an area is thus a very unpredictable parameter. The fibrotic tissue creates a barrier between the implanted sensor device and the tissue containing the analytes of interest, thereby causing for example a measurement delay at the sensor. Furthermore, the measurement may become additionally challenging as some analytes may more readily pass the barrier than others.

Formation of the fibrotic tissue under response of a healthy immune system typically takes approximately 14 - 20 days. One way to address this issue is therefore to regularly remove the implanted sensor device and re-implant a sensor device in a different area. As a further measure, the need for this step can be delayed by supressing the body's immune response, for example by releasing immunosuppressive drugs (e.g. glucocorticosterioids like dexamethasone) from the implanted sensor device. Nevertheless, while this approach delays or blocks fibrotic tissue formation, vascularization is equally delayed or blocked. Moreover, the drug release is limited in time and replacement of the implanted sensor device is still necessary every couple of months.

There is thus still a need within the art for sensor devices which address some or all of the issues outlined above.

### Summary of the invention

It is an object of the present invention to provide good sensor systems and devices which calibrate to and/or modify a sensing environment. The above objective is accomplished by system devices according to the present invention.

It is an advantage of embodiments of the present invention that measured analyte concentrations can be calibrated for in situ determined environmental variables, such that a more accurate measurement value can be obtained.

It is an advantage of embodiments of the present invention that the calibration elements (processor and second integrated sensor) are part of the integrated sensor system, thus allowing automated and automatic calibration, without requiring much or any action from the user (although calibration or re-calibration may be initiated by a user or medical assistant). It is a further advantage of embodiments of the present invention that calibration can be done as often as required, e.g. at fixed time intervals, on demand, at moments in time defined by the system and depending on previous calibration results, etc.

It is an advantage of embodiments of the present invention that a plurality of environmental variables can be determined.

It is an advantage of embodiments of the present invention that angiogenesis in the sensing area can be stimulated. It is a further advantage of embodiments of the present invention that the analyte concentration measurement is thereby improved (e.g. the response time and/or the signal-to-noise ratio).

It is an advantage of embodiments of the present invention that the elements which allow the measurement of the environmental variable can also be used to stimulate angiogenesis.
In a first aspect, the present invention relates to a sensor system, comprising an integrated sensor device, comprising
a first integrated sensing element, configured for sensing a concentration of an analyte and a second integrated sensing element, configured for sensing an environmental variable; and
means for receiving and processing data, optionally partially or completely comprised in the integrated sensor device, and programmed for receiving
an analyte concentration signal from the first integrated sensing element, and
an environmental variable signal from the second integrated sensor element; wherein the means for receiving and processing data is programmed for calibrating an interpretation of the received analyte concentration signal based on the received environmental variable signal.
Where in embodiments of the present invention reference is made to an environmental variable signal, reference may be made to an environmental variable having an effect on the diffusivity of the analyte of interest or having an effect on angiogenesis. The environmental variable may for example be any or a combination of temperature, humidity, chemical composition of the environment, etc.
In a second aspect, the present invention relates to an implantable integrated sensor device for use in in vivo stimulation of angiogenesis.
Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a schematic representation of an integrated sensor device for measuring an environmental variable and implanted in a living tissue, in accordance with embodiments of the present invention.
Fig. 2 is a schematic representation of an integrated sensor device for stimulating angiogenesis and implanted in a living tissue, in accordance with embodiments of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element with the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The following terms are provided solely to aid in the understanding of the invention.

As used herein, and unless otherwise specified, integrated elements are elements integrate in the same sensor device. Similarly, an integrated device is a device consisting of components (e.g. elements and layers, including encapsulation structures) which are integrated in the same device.
In a first aspect, the present invention relates to a sensor system. The sensor system comprises an sensor device comprising a first integrated sensing element, configured for sensing a concentration of an analyte and a second integrated sensing element, configured for sensing an environmental variable. The sensor system also comprises a means for receiving and processing data, optionally partially or completely comprised in the integrated sensor device, and programmed for receiving an analyte concentration signal from the first integrated sensing element, and an environmental variable signal from the second integrated sensor element. The means for receiving and processing data is, according to embodiments of the present invention, programmed for calibrating an interpretation of the received analyte concentration signal based on the received environmental variable signal.
The sensor system of the present invention may be particularly useful as a biosensor system for measurements in a living being; as such, exemplary features of the present invention are disclosed throughout this application pertaining to this application area. Nevertheless, it will be appreciated that the invention is not limited thereto. Indeed the sensor system may for example also be useful in many other technological areas, for example in waste collection where there can be a desire for the in situ monitoring of toxins or other harmful substances in a recipient. The present invention can be used to account for e.g. the buildup of a deposit which limits diffusion of the analyte towards the sensing element, or an environmental change.

In embodiments, the integrated sensor device may be an implantable integrated sensor device. When implanted, the integrated sensor has the advantage that analyte concentrations may be monitored continuously and automatically, avoiding the need for a recurrent interventional method (e.g. pricking of the finger in glucose monitoring). In embodiments, the implantable integrated sensor may comprise an exterior which is bio-compatible. In preferred embodiments, the integrated sensor device may be implanted subcutaneously. For subcutaneous implantation, the invasive placement of the integrated sensor device advantageously typically constitutes only an uncomplicated, low-risk medical intervention. In other embodiments, the integrated sensor device may be implanted percutaneously. Percutaneous implantation typically involves implantation into another organ below the skin, making it typically a higher risk, highly invasive medical intervention.

In embodiments, the environmental variable may be a hydration level. A sensor system according to embodiments of the present invention may comprise the sensing of a hydration level of an environment (e.g. of a tissue in contact with the integrated sensor device). The hydration level is an external, environmental variable that influences the accurate calibration of a measured analyte concentration (e.g. an accurate determination of the blood or body sugar level). As an efficient diffusion medium for analytes, the amount of fluid (e.g. interstitial fluid) has a direct impact on the analyte concentrations measured by the first sensing element. These analyte concentrations measured in the sensing environment may vary significantly from those concentrations in the desired reference environment (e.g. blood); hence the need for calibration of the measured concentration. The hydration level may also have an impact on the permeability of the environment, which determines the uptake of analytes towards the sensing environment (e.g. from small blood vessels towards the interstitial fluid). It is therefore an advantage of embodiments of the present invention to collect additional, local information on the tissue hydration level as an input for an algorithm that recalibrates the measured analyte concentration levels as a function of the degree of tissue hydration. In embodiments, calibrating an interpretation of the received analyte concentration based on the received environmental variable may comprise for example using a model for the interpretation of the analyte concentration as a function of the hydration level, or using a look up table of the interpretation of the analyte concentration based on the hydration level.

In embodiments, the environmental variable may be representative of a level of fibrotic tissue, such as an amount, a density and/or a quality of fibrotic tissue. The formation of fibrotic tissue is a consequence to the body responding to the introduction of a foreign object and is part of the wound healing mechanism. As outlined in the background of the invention, the fibrotic tissue presents a barrier for the analyte, having a direct influence on the diffusion properties of the analyte and delaying the integrated sensor device's response to analytes diffusing from the blood vessels through the tissue to the first sensing elements of the integrated sensor device. It is therefore an advantage of embodiments of the present invention that the formation of a fibrotic tissue adjacent to the implanted integrated sensor device can be monitored and calibrated for. It is a further advantage that this can also allow to confirm a normal wound healing process after the invasive intervention. As the fibrotic tissue thickness and density may vary from patient to patient in an unpredictable way, depending on the patient's body immune response, the specific tissue location within the body, the size, the material and orientation of the integrated sensor device, etc., the changes in fibrotic tissue formation are advantageous regularly detect and account for. In embodiments, calibrating an interpretation of the received analyte concentration based on the received environmental variable may comprise for example using a model for the interpretation of the analyte concentration as a function of the level of fibrotic tissue, or using a look up table of the interpretation of the analyte concentration based on the level of fibrotic tissue.

In a first type of embodiments, the second sensing element may comprise a resonant electronic circuit, the resonant electronic circuit comprising at least one electronic element which is exposed to the environment. In particular embodiments, an adjacent tissue may be a component of the electronic resonant circuit. Upon a change of the environmental variable in the sensing area, e.g. a change in hydration level of the tissue or the amount or quality of fibrotic tissue, the impedance of the tissue may typically change accordingly. This leads to a detectable shift in the electronic resonant circuits which provides additional input to a calibration algorithm, which may accounts for the changing environment variable and accordingly adjust the interpretation of the measured analyte concentration.

In a second type of embodiments, the second sensing element may comprise an electronic circuit adapted for measuring an impedance. In embodiments, measuring the impedance may comprise an electrical impedance spectroscopy. In embodiments, the impedance may be characterized by applying voltages to electrodes in contact with the environment (e.g. adjacent tissue) and measuring electric currents traversing the environment. In embodiments, the electronic circuit adapted for measuring an impedance may comprise a four-wire sensing. The four-wire sensing of an impedance is advantageously known to be very accurate; it typically involves applying a voltage between an electrode pair and detecting a current by a different electrode pair, or the same electrode pair with 4 independent wires. In embodiments, measuring the impedance may comprise using a plurality of electrode pairs. Expanding the four-wire sensing technique to use a plurality of voltage applying electrode pairs and a plurality of current detecting electrode pairs advantageously allows an electric impedance tomography to be performed, providing more detailed 3D information of the environment (e.g. properties of the adjacent tissue).

The electrode material of one or multiple electrodes can also be chosen to be capable of measuring other environmental parameters such as the pH (e.g. Iridium Oxide, conductive polymers such as PEDOT or carbon-based electrodes) and the electrodes and the integrated electronics can be configured to do potentiostatic or ampereostatic analyses of the environment in e.g. a 3-, or 4-electrode set-up, allowing an electrochemical study of the environment.

The second sensing element also may be adjusted to measure a voltage or a current and plotting other collected data (e.g. imaginary part vs real parts of the impedance) to differentiate between analytes. The collected data can be represented for example in Nyquist, Bode or Warburg plots.

In a third type of embodiments, the second sensing element may be adapted for measuring an osmotic action. In embodiments, the integrated sensor device may comprise at least one encapsulation structure. An integrated sensor device according to embodiments of the present invention may comprise an encapsulation material for encapsulating the sensor device. Several types of encapsulation may be provided for. A first type of encapsulation structure may comprise a hermetic packaging. The hermetic packaging insulates electronic circuits in the integrated sensor device from a humid environment and thereby avoids failure of the electronics. A second type of encapsulation structure may comprise a soft encapsulant (620) (e.g. a polymer). The soft encapsulant (620) may protect the hermetic seal and/or the substrate from corrosion (e.g. due to the wet environment). A better shielding and insulation of sensitive components in the device is thus achieved by the first and/or second type of encapsulation. For example, body fluids may be prevented from reaching sensitive electronic components and vapor condensation taking place on sensitive electronic components is avoided, improving their long-term reliability. A third type of encapsulation structure may comprise a thin layer that enhances tissue restoration and/or inhibits scar tissue formation. The thin layer may for example comprise a thin pharmaceutical coating or an engineered material (e.g. a mesh or scaffold). This encapsulation facilitates wound healing and thereby improves the incorporation of the integrated sensor device into the connective tissue.

In embodiments, the means for processing and receiving data may comprise a part which is comprised in the integrated sensor device (i.e. an internal part) and a part which is not comprised in the integrated sensor device (i.e. an external part). In embodiments, each part may comprise a microprocessor. In embodiments, the integrated sensor device may comprise at least a part of the means for processing and receiving data that is hermetically shielded from the environment. An integrated sensor device according to embodiments of the present invention may comprise a hermetically insulated housing for the part of the means for processing and receiving data comprised therein (i.e. the internal part), e.g. a hermetically sealed housing comprising a microprocessor.

In embodiments, the analyte may be glucose, urea, creatinine, triglycerie, protein, cholesterol, ethanol, ketones, hormones or lactate. In embodiments, the analyte may be a biomarker. The level of glucose concentration in blood (i.e. the blood sugar level) is for example an important indicator for diabetes and can be used to determine the need for an injection of insulin. It is therefore an advantage for persons suffering from diabetes to have a reliable, continuous glucose level monitoring sensor device in order to provide accurate medical follow up and thus prevent complications. Similarly, accurate measurements of urea, lactate, ketones and/or may also lead to a more accurate medical follow up of a patient. In embodiments, the analyte may also be a toxin, pharmaceuticals, a radioactive species, or any other analyte that may be of interest.

The first sensing element may typically be any sensing element capable of measuring (or detecting) the analyte. In embodiments, the first sensing element may comprise an electrochemical sensing element. In embodiments, the first sensing element may comprise an optical transducer. In embodiments, the optical transducer may comprise an optical waveguide element coated with biomarkers, or optical coupling elements or evanescent sensing waveguides. The first sensing element also may be adapted for performing colorimetry.

In embodiments, the integrated sensor device may be made in silicon or in a silicon compound. In embodiments, the silicon compound may be selected from a silicon nitride, a silicon oxide, a silicon carbide or a silicon oxynitride. This advantageously means that well-established silicon chip fabrication methods may be used to manufacture the integrated sensor device, making it cost-effective and mass-producible.

In embodiments, any feature of any embodiment of the first aspect may independently be as correspondingly described for the second aspect and its embodiments.

In a second aspect, the present invention relates to an implantable integrated sensor device for use in in vivo stimulation of angiogenesis.
The formation of blood vessels (i.e. angiogenesis) in closer proximity to the implanted integrated sensor device reduces the diffusion distance of the analytes and therefore increases their uptake and detected concentration levels. The implanted integrated sensor device's signal-to-noise ratio and recalibration capabilities are thereby advantageously improved. Once the blood vessels are formed they typically do not quickly deteriorate. As such, it is advantageously typically sufficient to promote angiogenesis during e.g. fibrous tissue formation, after which the improvements in the sensing area are maintained relatively indefinitely.
In embodiments, the implantable integrated sensor device comprises at least one sensing element and an angiogenesis stimulating element.
In a first type of embodiments, the angiogenesis stimulating element may comprise a conformal porous coating surrounding the at least one sensing element. In embodiments, the conformal or local porous coating may be selected from an electrospun material, a fabric or a surgical mesh.
In a second type of embodiments, the angiogenesis stimulating element may comprise a plurality of electrodes adapted for generating an electric field. In embodiments, the electric field may extend into a tissue when implanted, the tissue being adjacent to the implantable integrated sensor. In embodiments, the electric field may be maintained for a predetermined time period. Without being bound by theory, and while further scientific discussion is still ongoing, it is currently believed that the stimulation of angiogenesis by an electric field is characterized by a simple model of the attraction of electrically charged vascular tissue growth factors towards one of the electrodes used to generate the electric field. A further use of the angiogenesis stimulating element may therefore also be that of attracting certain analytes (e.g. charged analytes) towards the sensing element.

In embodiments, the angiogenesis stimulating element may be within 100 µm from the sensing element, preferably within 10 µm, yet more preferably within 1 µm. The angiogenesis stimulating element is preferably relatively (e.g. in the direct vicinity) of the sensing element, so that vascular tissue is formed within the sensing area.

In embodiments, any feature of any embodiment of the second aspect may independently be as correspondingly described for the first aspect and its embodiments. In this respect, the sensing element of the second aspect typically corresponds to the first sensing element of the first aspect.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

### Example 1: Sensor system comprising calibration based on a level of fibrotic tissue

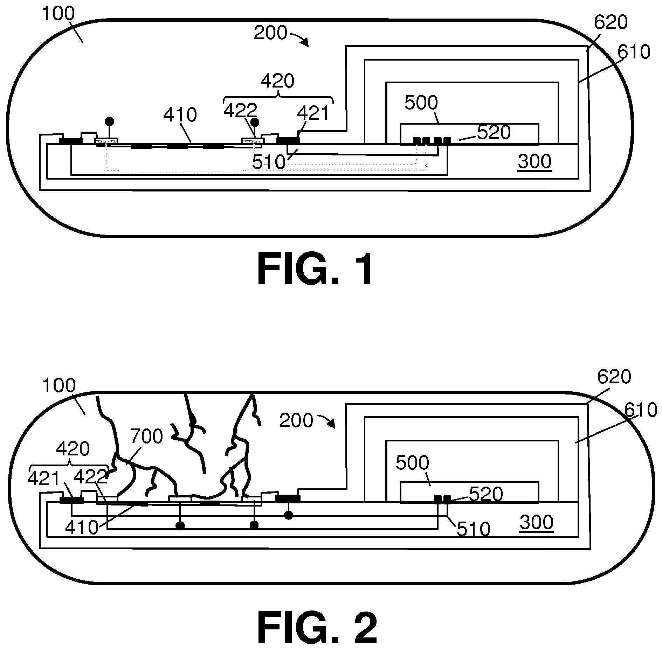

We now refer to Fig. 1, schematically showing an integrated sensor device 200 implanted in living tissue 100 (e.g. a subcutaneous tissue of a human being). The integrated sensor device 200 is part of a sensor system which further comprises an external (i.e. not implanted) means for receiving and processing data (not shown) received from the integrated sensor device 200. The integrated sensor device 200 comprises a first integrated sensing element 410 and a second integrated sensing elements 420 on a substrate 300. The first integrated sensing element 410 is configured for measuring an analyte (e.g. glucose, urea, creatinine, triglycerie, protein, cholesterol, ethanol, ketones, hormones or lactate) concentration, for example based on an optical, electrochemical or enzymatic sensing. The second sensing element 420 is configured for measuring a level of fibrotic tissue in the sensing area, and may for example comprise one or more electrode pairs which are in contact with the living tissue 100. In this example, a 4-wire impedance measurement is implemented to measure the impedance of the tissue 100: a first electrode pair 421 generates a low amplitude current through the tissue 100, while a second electrode pair 422 measures the voltage drop across the tissue 100. Through individual electrical lines 510, each electrode is coupled to an electronic circuit 520 which steers the measurement. The electrical lines 510 run embedded in the substrate 300 and are insulated from each other. The integrated sensor device 200 further comprises a hermetically sealed chamber 610 which houses electronic circuitry 500 for the integrated sensor device 200 and protects it from corrosion, for example comprising electronics for driving the first integrated sensing element 410 and the second integrated sensing element 420 and for receiving signals therefrom, as well as further electronic units for driving the device and for communicating to the external means for receiving and processing data, etc. The means for receiving and processing data may thus be thought of as being split in two parts: an internal part 520, typically comprised in the hermetically sealed chamber 610 and e.g. performing a pre-processing of the data, and an external part (not shown), outside the body and e.g. further processing the received pre-processed data (for example enabling said data to be displayed to a user in a useful format). The integrated sensor device 200 is further encapsulated in a soft encapsulant 620 (e.g. a polymer) with openings therein for the sensing elements 410 and 420.

The means for receiving and processing data is adapted to use the impedance measured by the second sensing element 420, i.e. a sensed environmental variable representative of the level of fibrotic tissue in the sensing environment, in order to interpret the analyte concentration measured by the first sensing element 410. In this way, the sensor system is able to calibrate its measurements of the analyte concentration, taking into account the amount and quality of fibrotic tissue that has formed, and is able to update this calibration as required.

### Example 2: Sensor system comprising calibration based on an environmental hydration system

The sensor system has the same characteristics as in example 1, but differs in that the second sensing element is configured for measuring a hydration level of the surrounding tissue. The hydration level measurement may again be based on an impedance measurement (e.g. a 4-wire impedance measurement). Alternatively the measurement may comprise the use of a resonant electronic circuit having an electronic element that is exposed to the surrounding environment; or it may comprise the use of an osmotic actuator whose response towards variations in the surrounding hydration (or electrolyte concentration levels) has a measurable mechanical or electrical response.

The sensor system is again able to calibrate its measurements of the analyte concentration, this time taking into account the hydration level of the sensing environment.

### Example 3: Implantable integrated sensor device for stimulating angiogenesis

We now refer to Fig. 2, schematically showing an integrated sensor device 200 implanted in living tissue 100 (e.g. a subcutaneous tissue of a human being). The integrated sensor device 200 comprises an integrated sensing element 410, configured for measuring an analyte (e.g. glucose, urea, creatinine, triglycerie, protein, cholesterol, ethanol, ketones, hormones or lactate) concentration, for example based on an optical, electrochemical or enzymatic sensing. The integrated sensor device 200 further comprises an angiogenesis stimulating element 420, in the form of one or more electrode pairs 420 in close vicinity to the integrated sensing element 410. An electrical field can be generated across the electrode pairs 420, which induces or stimulates formation of vascular tissue 700 (i.e. angiogenesis) in the surrounding tissue 100. Through individual electrical lines 510, each electrode is coupled to an electronic circuit 520 which manages the electrodes. The electrical lines 510 run embedded in the substrate 300 and are insulated from each other. The integrated sensor device 200 further comprises a hermetically sealed chamber 610 which houses electronic circuitry 500 for the integrated sensor device 200 and protects it from corrosion, for example comprising electronics for driving the integrated sensing element 410 and the angiogenesis stimulating element 420 and for receiving signals therefrom, as well as further electronic units for driving the device and for communicating to the external means for receiving and processing data, etc. The integrated sensor device 200 is further encapsulated in a soft encapsulant 620 (e.g. a polymer) with openings therein for the sensing elements 410 and 420.

In this way, the sensor system is able to modify the sensing environment by stimulating the vascular growth therein. As such, the analyte can traverse any barrier formed by the fibrotic tissue through the blood vessels that penetrate the fibrotic tissue; thereby mitigating the effects of the fibrotic tissue formation. To this end, the angiogenesis stimulating element 420 may for example be utilized throughout the healing process of the body after the medical intervention (i.e. throughout the period of fibrotic tissue formation after implantation of the integrated sensor device 200).

Another application of the angiogenesis stimulating element 420 (i.e. the electrode pairs 420) may be to attract certain biomarkers of interest (e.g. analytes) towards the sensing area, based on charge transport across the electrical field.

### Example 4: Sensor system combining the functions of examples 1 to 3

A sensor system as described in example 1 may advantageously combine the functions as described in examples 1 to 3. To this end, after the implantation of the sensor device, one or more of the electrode pairs may be used as angiogenesis stimulating element, for example until formation of the fibrotic tissue is completed (e.g. roughly after 2 weeks). Simultaneously, for example intermittently, the electrode pairs can be used as second sensing element to measure the impedance of the surrounding tissue and thereby track the fibrotic tissue formation. Furthermore, e.g. after the fibrotic tissue formation is complete, these electrode pairs can also be used to measure the hydration level of the surrounding tissue; again based on an impedance measurement.

It is to be understood that although preferred embodiments, specific constructions and configurations, as well as materials, have been discussed herein for devices according to the present invention, various changes or modifications in form and detail may be made without departing from the scope and technical teachings of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A sensor system, comprising at a least
i. an integrated sensor device (200), comprising
- a first integrated sensing element (410), configured for sensing concentration levels of an analyte and
- a second integrated sensing element (420), configured for sensing an environmental variable; and
ii. a means for receiving and processing data being programmed for receiving
- an analyte concentration signal from the first integrated sensing element (410), and
- an environmental variable signal from the second integrated sensing element (420);
wherein the means for receiving and processing data is programmed for calibrating the received analyte concentration signal based on the received environmental variable signal.

2. The sensor system according to claim 1, wherein the integrated sensor device (200) is an implantable integrated sensor device (200).

3. The sensor system according to any of the previous claims, wherein the second sensing element (420) comprises a resonant electronic circuit, the resonant electronic circuit comprising at least one electronic element which is exposed to the environment.

4. The sensor system according to any of the previous claims, wherein the second sensing element (420) comprises an electronic circuit adapted for measuring a voltage, a current and for calculating an impedance.

5. The sensor system according to claim 4, wherein the electronic circuit adapted for measuring a voltage, a current and calculating an impedance comprises four-wire sensing.

6. The sensor system according to any of the previous claims, wherein the first sensing element (410) comprises an optical waveguide element coated with biomarkers, optical coupling elements or evanescent sensing waveguides.

7. The sensor system according to any of the previous claims, wherein the environmental variable is a hydration level.

8. The sensor system according to claim 7, wherein calibrating an interpretation of the received analyte concentration based on the received environmental variable comprises:
- using a model for the interpretation of the analyte concentration as a function of the hydration level, or
- using a look up table of the interpretation of the analyte concentration based on the hydration level.

9. The sensor system according to any of claims 1 to 6, wherein the environmental variable is representative for a level of fibrotic tissue.

10. The sensor system according to claim 9, wherein calibrating an interpretation of the received analyte concentration based on the received environmental variable comprises:
- using a model for the interpretation of the analyte concentration as a function of the level of fibrotic tissue, or
- using a look up table of the interpretation of the analyte concentration based on the level of fibrotic tissue.

11. The sensor system according to any of the previous claims, wherein the integrated sensor device (200) comprises at least one encapsulation structure (610,620).

12. The sensor system according to any of the previous claims, wherein the integrated sensor device (200) comprises at least a part (520) of the means for processing and receiving data that is hermetically shielded (610) from the environment.

13. The sensor system according to any of the previous claims, wherein the analyte is one or more of glucose, urea, creatinine, triglycerie, protein, cholesterol, ethanol, ketones, hormones or lactate.

14. An implantable integrated sensor device (200) for use in in vivo stimulation of angiogenesis.

15. The implantable integrated sensor device (200) according to claim 14, comprising at least one sensing element (410) and an angiogenesis stimulating element (420).
